(19) Europäisches Patentamt
European Patent Office
Office européen des brevets

(11) Publication number: **0 088 845**
**A2**

# (12) EUROPEAN PATENT APPLICATION

(21) Application number: 82305399.6

(22) Date of filing: 11.10.82

(51) Int. Cl.³: C 08 F 220/28
C 08 F 220/32, C 08 L 33/14
A 61 F 11/04, A 61 C 5/00
A 61 C 13/08, A 61 K 6/08
A 61 F 1/16, G 02 B 1/02

(30) Priority: 12.10.81 GB 8130758

(43) Date of publication of application:
21.09.83 Bulletin 83/38

(84) Designated Contracting States:
CH DE LI SE

(71) Applicant: **Secretary of State for Social Services in Her Britannic Majesty's Government of the United Kingdom of Great Britain and Northern Ireland Alexander Fleming House Elephant & Castle
London SE1 6BY(GB)**

(72) Inventor: **Bhusate, Mangala
62 Brookside Road
Golders Green London NW11 9NG(GB)**

(72) Inventor: **Braden, Michael
26 Stewart Road
Harpenden Hertfordshire AL5 4QB(GB)**

(74) Representative: **Gunning, William John et al,
Procurement Executive, Ministry of Defence Patents
1A(4), Room 2014 Empress State Building Lillie Road
London SW6 1TR(GB)**

## (54) Low shrinkage polymers and process for their preparation.

(57) A process for the preparation of a polymeric material that has a linear curing shrinkage of 1% or less involves mixing a liquid component containing, as its sole polymerisable constituent, one or more monomeric esters of general formula I

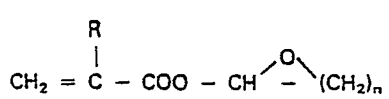

I

wherein R is a hydrogen atom or a methyl radical and n is 1, 2, 3 or 4, with a solid component containing an acrylate polymer, a methacrylate polymer or a copolymer of these polymers, and initiating the polymerisation of the one or more monomeric esters in the liquid component. Preferred materials are tetrahydrofurfuryl methacrylate in the liquid component and polyethyl methacrylate in the solid component. The polymerisation is preferably initiated by benzoyl peroxide in the presence of a chemical activator, especially *N, N* – dimethyl – *p* – toluidine.

The polymeric materials produced are particularly suitable for use in the formation of biomedical appliances, such as hearing aids, tooth filling, dental bridges and crowns, false teeth, artificial eyes and contact lenses, or for use in other biomedical applications, for example as a bone cement.

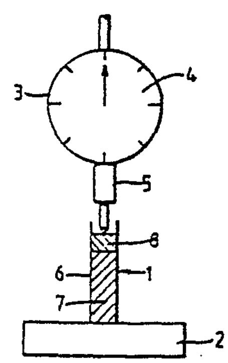


*Fig. 1*

EP 0 088 845 A2

LOW SHRINKAGE POLYMERS

The present invention relates to polymers that have a low linear shrinkage during the room temperature polymeric curing process and, in particular, to low shrinkage polymers that are useful for biomedical use.

The term "biomedical" as used hereinafter encompasses the medical (including aural), opthalmic, surgical, dental and related fields, whilst the term "biomedical appliance" refers to an appliance, such as a hearing aid, articificialeyes, contact lenses, a tooth filling or crown, dentures or a bone cement, that may be used in one or more of these fields.

When a polymeric material is employed in a situation requiring dimensional accuracy, for example in dental and hearing aid applications, one of the most important properties to be considered is its dimensional stability.

The particular synthetic resin most used at present in the dental and hearing aid fields is poly(methyl methacrylate). In a typical molding procedure, this polymer, in powdered form, is first mixed with its liquid monomer, methyl methacrylate. The monomer partially dissolves the polymer to form a plastic dough which is then packed into a suitable mold. Finally the monomer is polymerised, by heat or chemical activation, to give a hard, transparent material.

When methyl methacrylate monomer is polymerised the density changes, from 0.94 to 1.19 gms per cc, resulting in a volumetric shrinkage of 21%. If the conventional powder-liquid process, outlined above, is used in the molding procedure, approximately one-third of the volume of the dough is monomor. Consequently the average volume shrinkage of the dough during the polymerisation step will be about 7%. This is equivalent to a linear curing shrinkage of between about 2 and 2½%.

Clearly such a high degree of shrinkage is undesirable since it may lead to errors being made in the molding process, producing, for example, ill fitting dentures and/or hearing appliances. There is therefore a need for a polymeric material that has a low linear curing shrinkage and, preferably, that may be used as a biomedical material, particularly in the dental and aural fields.

According to the present invention there is provided a process for the preparation of a polymeric material suitable for biomedical use and having a linear curing shrinkage of 1% or less which comprises mixing a liquid component which contains, as its sole polymerisable constituent, one or more monomeric esters of general formula I

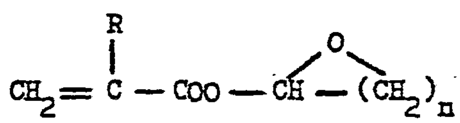

I

wherein R is a hydrogen atom or a methyl radical and n is 1,2,3 or 4, with a solid component which comprises one or more polymers selected from the group consisting of an acrylate polymer, a methacrylate polymer and a copolymer of these polymers, and initiating the polymerisation of the one or more monomeric esters in the liquid component.

Preferably the linear shrinkage of the polymeric material is between 0.2 and 1%. Moreover, in preferred embodiments of the present process the monomeric esters are methacrylate ($R = CH_3$) esters wherein n is 1, 2 or 3, with tetrahydrofurfuryl methacrylate ($R = CH_3$, n = 3) being particularly preferred.

Although a mixture of two or more monomeric esters may be added to the polyacrylate,polymethacrylate or copolymeric esters in the solid component to form a polymeric material according to the present invention, the addition of only one monomeric ester, particularly tetrahydrofurfurylmethacrylate, is preferred.

The acrylate or methacrylate ester polymers that are contained in the solid component may be in either homopolymeric of copolymeric form. Preferably the polymeric esters have a softening point (Tg) above about 50°C. In particularly preferred emobodiments of the process of the present invention the polymeric esters are selected from the group consisting of polymethyl, polyethyl, polypropyl or polybutyl methacrylate or copolymers of these polymers. Polyethyl methacrylate and copolymers of this polymer with polymethyl, polypropyl and polybutyl methacrylate are especially preferred.

Although a mixture of two or more polymeric esters may be admixed with the monomeric acrylate of methacrylate esters to form a polymeric material according to the present invention, the use of only one polymeric ester, particularly polyethyl methacrylate is preferred.

In another aspect of the present invention the polymeric esters in the solid component, prior to admixing with the monomeric esters in the liquid component, may be admixed with up to 10% (by wt) of an inorganic filler, such as finely-divided alumina silicates, silica, quartz, glass, and the like. However, if an inorganic filler is used it is important that the physical and chemical properties of the polymeric material produced do not differ significantly from the properties of the equivalent inorganic filler free polymeric material.

On mixing the polymers of the solid component and monomers of the liquid component, the polymerisation of the monomers in the liquid component may be initiated by any of the polymerisation initiators that are well knownin the polymer art. Typically, however, free radical catalysts, such as the organic peroxides, benzoyl peroxide and lauroyl peroxide, are used. Often a residual amount of the peroxides may be present in the polymer of the solid component. If this is the case it may not be necessary to add any more of the catalyst. On the other hand if there is not sufficient residual peroxide present, it should be added, preferably to the solid component, until a suitable concentration is present. The initiators, particularly benzoyl peroxide, may be activated by using heat. Preferably, however, activation is effected with the aid of a chemical activator, so that the polymerisation may be completed at room temperature (so-called "cold curing"). Preferred activators include organic tertiary amines, such as _N_, _N_ - dialkyl - _p_ - toluidines, _N_, _N_ -dialkyl anilines and _N_ - _p_ - tolyl - dialkanolamines. In particular preferred embodiments of the process of the present

BAD ORIGINAL

invention the activator is selected from the group consisting of N, N - di methyl-p-toluidine, N, N - diethyl-p-toluidine, N, N - dimethylaniline and N-p-toluene-diethanolamine. Preferably the chemical activator is present in the liquid component prior to mixing.

Other conventional initiators and activators, while not at present preferred, may be used in place of or together with the above substances, provided they lead to an efficient polymerisation step. Further additives, such as pigments, to produce a coloured material, and stabilising agents (for example sulphinic acid derivatives, especially p - toluenesulphinic acid), to prevent oxidation of the tertiary amines and thereby to stabilise the colour of the resultant polymeric material, may be present in the initial reaction mixture.

The ratio of polymer in the solid component to monomer in the liquid component that is used to form the preferred polymeric material of the present invention is determined by a number of factors. In general, the more polymer that is used, the less will be the reaction time between the polymer and the monomer. By contrast, the resin will tend to shrink less during processing if less monomer is used. On the other hand, sufficient monomer must be employed to wet the polymer thoroughly, the choice of polymer/monomer ratio is therefore one of compromise. In a preferred embodiment of the present invention the polymer/monomer ratio is between 3 to 1 and 1.5 to 1 by weight.

The formation of the polymeric material in the process of the present invention by mixing ester polymers with ester monomers in the presence of initiators and activators is characterised by four distinct stages.

1. On mixing polymer with monomer a fluid, incoherent mass is formed.
2. The mixture then becomes tacky and, if pulled apart, exhibits a fibrous nature.
3. The mixture loses its tacky quality and becomes smooth and dough like. This is referred to as the dough or gel stage.
4. The mass becomes cohesive and rubber-like.

The time required to reach stage 3 depends on the solubility of the polymer in the monomer. This time will depend on a number of factors, but expecially on the temperature of the reaction and the size of the polymer particles. Generally, the higher the temperature and the smaller the particle size, the faster stage 3 will be reached. In a preferred

embodiment of the present invention the reaction to reach the dough stage is conducted at a temperature of between about 15 and 40°C whilst the mesh size of the ester polymer is between about 200 and 300.

It is during stage 3 that the preferred polymeric material of the present invention is molded into a required shape, particularlythat of a biomedical appliance. For the manufacture of hearing aids, dentures, tooth fillings and crowns, it is preferred that the dough stage 3 should last at least 5 minutes. In order to achieve this with the preferred materials of the present invention, using a polymer with a mesh size of 200 to 300, the reaction should be conducted at a temperature of between about 15 and 40°C, preferably between about 20 and 25°C.

The present process will be used predominantly in the biomedical area, for example in dental surgeries and laboratories or in medical laboratories. In order to facilitate the use of the process in such areas there is also provided, in a further aspect of the present invention, a kit for use in the preparation of a polymeric material comprising a liquid component which contains, as its sole polymerisable constituent, one or more monomeric esters of general formula I

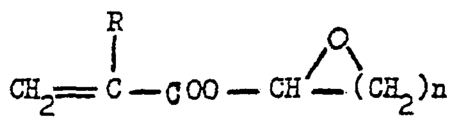

I

wherein R is a hydrogen atom or a methyl radical and n is 1, 2, 3 or 4, and a solid component which comprises one or more polymers selected from the group consisting of an acrylate polymer, a methacrylate polymer and a copolymer of these polymers.

In preferred embodiments of the present kit the monomeric esters are methacrylate ($R = CH_3$) esters wherein n is 1, 2 or 3, with tetrahydrofurfuryl methacrylate ($R = CH_3$, $n = 3$) being particularly preferred.

Although a mixture of two or more monomeric esters may be used in the liquid component of the present kit, it is preferred that the liquid component contains only one monomeric ester, preferably tetrahydrofurfuryl methacrylate.

The acrylate or methacrylate ester polymers that are contained in the solid component of the present kit may be in either homopolymeric or copolymeric form. Preferably the polymeric esters have a softening point (Tg) above about 50°C. In particularly preferred embodiments of the present kit the polymeric esters are selected from the group consisting of polymethyl, polyethyl, polypropyl or polybutyl methacrylate or copolymers of these polymers. Polyethyl methacrylate and copolymers of

BAD ORIGINAL

this polymer with polymethyl, polypropyl and polybutyl methacrylate are especially preferred.

Although a mixture of two or more polymers may be used in the solid component of the present kit, it is preferred that the solid component contains only one polymeric ester, preferably polyethyl methacrylate.

The present kit may further comprise an inorganic filler and a polymerisation initiator. The inorganic filler, which is generally in admixture (up to 10% by wt) with the one or more polymers of the solid component, may be chosen from any of the inorganic fillers that are well known in the polymer art. Examples include finely-divided alumina silicates, silica, quartz and glass. However if an inorganic filler is used it is important that the physical and chemical properties of the polymeric material finally produced do not differ significantly from the properties of the equivalent inorganic filler free polymeric material.

The polymerisation initiator may also be any of those that are well known in the polymer art. Typically, however, free radical catalysts, such as the organic peroxides, benzoyl peroxide and lauroyl peroxide, are used. Often a residual amount of the peroxides may be present in the polymer of the solid component. If this is the case it may not be necessary to add any more of the catalyst to the kit. On the other hand if there is not sufficient residual peroxide present, it should be added, preferably to the solid component, until a suitable concentration is present. The initiators, particularly benzoyl peroxide, may be activated by using heat. Preferably, however, activation is effected with the aid of a chemical activator, so that the polymerisation may be completed at room temperature (so-called "cold curing"). If this "cold curing" is the process of choice th n the present kit may also comprise a chemical activator for the polymerisation initiator. Preferred activators include organic tertiary amines, such as N, N - dialkyl- p - toluidines, N, N - dialkylanilines and N - p - tolyl - dialkanolamines. In particularly preferred embodiments of the kit of the present invention the activator is selected from the group consisting of N, N - dimethyl-p-toluidine, N, N - dimethylaniline, N - p - toluene-diethanolamine and N,N - diethyl - p - toluidine. Preferably the chemical activator is present in the liquid component of the kit.

Other conventional initiators and activators, while not at present

preferred, may be used in place of or together with the above substances, provided they lead to an efficient polymerisation step. Further additives, such as pigments, to produce a coloured material, and stabilising agents (for example sulphinic acid derivatives, especially p - toluenesulphinic acid), to prevent oxidation of the tertiary amines and thereby stabilise the polymer's colour, may also be present in alternative embodiments of the present kit. Moreover, in order to facilitate the use of the present kit in the present process, a set of instructions, giving process details, may also be included in a preferred form of the present kit.

The polymeric materials prepared by the process of the present invention are suitable for use in any area where a resinous or hard plastics material with low linear curing shrinkage is required. However, the present materials are particularly suitable for use in the formation of biomedical appliances, such as hearing aids, tooth fillings, dental bridges and crowns, dentures, artificial eyes and contact lenses, or for use in other biomedical applications, for example as a bone cement.

The polymeric materials, processes of forming the polymeric materials and applications for the polymeric materials will now be described by way of example only with particular reference to the figures in which:

Figure 1 shows a polymerisation shrinkage apparatus; and

Figure 2 shows the linear polymerisation shrinkage of a number of acrylic resins against time.

Example 1a

Formation of the polymeric material

A polymeric material was prepared in accordance with the present invention wherein the solid phase was composed of 99 parts by weight of 240 mesh size polyethyl methacrylate and 1 part by weight of benzoyl peroxide. The liquid portion of the composition was prepared by mixing 97.5 parts by weight of tetrahydrofurfuryl methacrylate and 2.5 parts by weight of N, N - dimethyl - p - toluidine.

The solid and liquid phases were then blended (in the ratio 1.0g powder to 0.624g liquid) at 20°C to form, after about 5 mins, a dough-like composition which, after a further 10 mins, had hardened to a solid resin.

b. The procedure of Example 1a was repeated except that the ratio of solid to liquid that was blended was 1.2g to 0.624g.

BAD ORIGINAL

c. The procedure of Example 1a was repeated except that the ratio of solid to liquid that was blended was 1.5g to 0.624g.

Example 2

Polymerisation shrinkage

The linear polymerisation shrinkage of the polymeric material produced in Example 1b and of two known materials was measured on the apparatus illustrated in Figure 1. Referring to Figure 1, the polymerisation shrinkage apparatus consists of glass tube 1 standing on a glass slab 2.

A dial guage 3 has a numbered face 4 and a freely movable indicating arm 5 that projects into the body of the glass tube 1 in parallel relation with the side walls 6 of the tube 1.

In use freshly mixed polymeric material 7 is introduced into the glass tube 1 and a plug 8 of the same material in a hardened state is placed on top of the freshly mixed polymer 7. The bottom of the indicating arm 5 is then put in contact with the plug 8 and as the freshly mixed material 7 hardens it also shrinks and thereby moves the plug 8 and the arm 5 further into the tube 1. The amount of linear shrinkage is indicated on the dial's numbered face 4.

The linear shrinkage of one of the polymeric materials of the present invention and two known acrylate polymers is illustrated in Figure 2.

The constituents of the three compositions are listed below.

Polyethyl methacrylate/Tetrahydrofurfuryl methacrylate (PEM/THM)

| | |
|---|---|
| Polyethylmethacrylate | 1.888 g |
| Benzoyl peroxide | 0.012 g |
| | |
| Tetrahydrofurfuryl methacrylate | 0.608 g |
| N,N - Dimethyl - p - toluidine | 0.016 g |

Polymethyl methacrylate/Methyl methacrylate (PMM/MM)

| | |
|---|---|
| Polymethyl methacrylate | 1.188 g |
| Benzoyl peroxide | 0.012 g |
| Methyl methacrylate | 0.608 g |
| N,N - Dimethyl - p - toluidine | 0.016 g |

Polyethyl methacrylate/n - Butyl methacrylate (PEM/BM)

| | |
|---|---|
| Polyethyl methacrylate | 1.188 g |
| Benzoyl peroxide | 0.012 g |
| n - Butyl methacrylate | 0.608 g |
| N,N - Dimethyl - p - toluidine | 0.016 g |

Example 3

Viscoelastic properties

The viscoelastic properties of the polymeric materials prepared by the methods of Examples 1a, 1b and 1c were measured by the torsional pendulum procedure described in M Braden et al, J Dent Res, 1968, 47, 519. The variation of shear modulus (real and imaginary, $G_1$ and $G_2$ respectively) and mechanical loss tangent (tan $\delta$) against temperature were plotted graphically to determine the Transition Temperature (Tg) of each polymeric material.

It was found that Tg increased with increasing Tetrahydrofurfuryl methacrylate content and that the viscoelastic properties were temperature dependent in the range 20-37°C.

Example 4

Mechanical properties

Rectangular strips of polyethyl methacrylate/tetrahydrofurfuryl methacrylate polymeric material (6 x 0.5 x 0.15 cm) were prepared according to the process of Example 1b. One end of each strip was clamped and, at the other end, the deflection (y) was measured with a travelling microscope for a series of added loads (W). Youngs Modulus (E) was calculated from the slope of the linear y-W relationship using the standard cantiliver beam formula: $y = \frac{3l^3gW}{Ebd^3}$; where l = the distance from the beam support to the point at which the load was applied, b = specimen width, d = specimen depth, g = acceleration due to gravity.

By this method the Young's Modulus of the polymeric material was found to be 1.0 GN/m$^2$.

Example 5

Water Absorption and Solubility

Specimens of the polymeric material having the polymer/monomer ratio of Example 1b were prepared in the form of a flat sheet. Each specimen was immersed in water at 37°C and weighed at suitable intervals until the absorption process was complete. The samples were then transferred to an air oven set at 37°C and weighing was continued during

BAD ORIGINAL

desorption until equilibrium was again established.

Classical diffision theory shows that for the early stages of water uptake, the ratio $Mt/M\infty$, ie the ratio of uptake at time t to the uptake at equilibrium, is given by, $Mt/M_\infty = 2(Dt/l^2)$. The thickness of the specimen is 2l, and D is the diffusion coefficient. $Mt/M_\infty$ plotted against $t^{\frac{1}{2}}$ is linear.

Similar conditions apply to desorption, except that Mt will refer to water loss.

The slope of the linear region that is obtained when $Mt/M_\infty$ is plotted against $t^{\frac{1}{2}}$ allow D(sorption) and D (desorption) to be calculated from the above equations. The diffusion coefficients

| | |
|---|---|
| Equilibrium uptake | : 2.2% |
| Diffusion Coefficient (sorption) | : $3.4 - 4 \times 10^{-8}$ $cm^2$ $sec^{-1}$ |
| (desorption) | : $9.5 - 10.3 \times 10^{-8}$ $cm^2$ $sec^{-1}$ |

Example 6

Polymerisation Exotherm

A sample of polyethyl methacrylate/tetrahydrofurfuryl methacrylate polymeric material was mixed according to one of the processes of Example 1a, 1b or 1c and put into a cylindrical cavity (1 cm diam x 1.1 cm high) drilled in a polytetraflouroethylene block. A copper/constantan thermocouple was then inserted centrally in the material. The thermocouple was connected to a potentiometric recorder (Smiths Servoscribe Record) so that the exotherm was plotted as a function of time.

The maximum change in temperature of the same ($\Delta T$) and the time taken to reach the maximum temperature (tmax) are given for various powder/monomer ratios in Table 1.

TABLE 1

| Powder/Monomer Ratio | $\Delta T$(°C) | $t_{max}$ (mins) |
|---|---|---|
| 1.0g/0.624g | 24.9 - 31.8 | 7.9 - 10.2 |
| 1.2g/0.624g | 24.0 - 29.2 | 6.5 - 8.6 |
| 1.5g/0.624g | 15.0 - 27.8 | 7.7 - 9.75 |

The range of results quoted arise from variable room temperatures.

Example 7

Preparation of a dental crown

A wax mould for a dental crown was prepared in conventional manner by the following procedure:-

a. formation of a model in stone;

b. wax-up of the desired form;

c. investment of model and waxed-up form; and

d. removal of wax form followed by cleaning.

The solid and liquid phases of Example 1b were then blended at 20°C to form a dough-like composition which was placed in the wax-mould and then allowed to polymerise. Finally, the moulded article was removed from the mould and ground and/or polished to produce the finished dental crown.

Example 8

Preparation of a dental bridge or denture

An impression of the indicated area was taken using a conventional impression material, such as sodium alginate. Liquid plaster of paris was then poured into the aforementioned impression and allowed to set. Upon the model so obtained a wax model of the appliance was formed. The colour, shape and correct bite of the teeth was then ascertained in conventional manner and the chosen teeth were set up on the wax denture, which was then invested in plaster. After the wax had been removed by boiling the teeth were reset in their original positions.

The solid and liquid phases of Example 1b were then blended at 20°C to form a dough-like composition, which was packed into the plaster denture in place of the wax, and then allowed to polymerise. Finally the cured article was removed from the plaster mould and ground and/or polished to produce the finished dental bridge or denture.

Example 9

Preparation of a dental filling

Cavities in teeth were prepared, in conventional manner, for receiving fillings. The solid and liquid phases of Example 1b were then blended at 20°C to form a dough-like composition. This composition was then placed in the prepared cavities and allowed to harden. Finally, the filling was ground and/or polished into the required form.

Example 10

Preparation of an ear mould for a hearing aid

A conventional impression material, such as silicone rubber, was inserted into the ear canal either by means of a syringe or by hand pressing. This process was continued until the canal, concha and helix were filled with impression material. After the impression material had hardened slightly it was removed from the ear and then invested in plaster.

The solid and liquid phases of Example 1b were then blended at 20°C to form a dough-like composition, which, after the impression material was removed, was packed into the plaster mould. After the resin had hardened, the article was removed from the mould and ground and/or polished into the required form.

Example 11

Preparation of a contact lens

A specimen of the polymeric material having the polymer/monomer ratio of Example 1b were prepared in the form of a rod with a 13 mm diameter. A blank was then turned from the rod and mounted with wax on a button which was, in turn, mounted in a lathe collet.

After the blank has been given a preliminary skim to remove the bulk of the unwanted material from its inside surface, it was mounted on a precision lathe set up for cutting a concave surface with a single-point diamond tool. The internal and external surfaces of the contact lens are then cut to provide the curve required for corneal contact. Finally, the concave and convex surfaces of the lens are polished on a polishing machine using a suitable polishing compound.

Example 12

Preparation of an artificial eye

A conventional impression material, such as silicone rubber, was inserted into the eye socket by hand pressing. After the impression material had hardened slightly it was removed from the socket and then invested in plaster. From this plaster model a wax pattern was produced. This pattern was invested in plaster in the shallow half of a dental crown flask, the plaster was trimmed and smoothed and then separating medium was applied. The counterpart was cast by vibrating plaster into both halves of the flask. The flask was then closed and the plaster allowed to set. After the plaster had set the flask was reopened and the pattern was removed. The plaster was then coated with a solution of an alginate.

The solid and liquid phases of Example 1b were then blended at 20°C to form a dough-like compositon, which, after further blending with the desired colouring substance (to produce the correct scleral colour), was packed into the flask. After the polymeric material had been cured, the flask was cooled and opened, and the acrylic sclera was removed.

The sclera was then drilled, filed and ground to produce a recess for the iris disc. After the iris disc had been cemented in the recess, the acrylic sclera was, if appropriate, further treated to match the patient's requirements. This further treatment may include the attachment

BAD ORIGINAL

of embroidery silk (to represent veins) to the sclera.

Finally the sclera was replaced in the dental crown flask, and dough-like material (prepared by the method of Example 1b) was gently pressed over its (the sclera's) surface to completely encapsulate it. The eye was again cured, and after cooling, removed from the flask. The back of the eye was polished with dental cones and cup brushes, whilst the front was abraded with cotton mops using lustre block as the polishing agent. The final polish was obtained with chamois leather mops.

BAD ORIGINAL

0088845 EPO

CLAIMS

1. A process for the preparation of a polymeric material suitable for biomedical use and having a linear curing shrinkage of 1% or less comprises
mixing a liquid component which contains one or more monomeric esters of general formula I

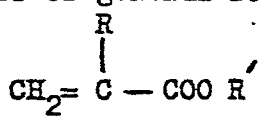

I

wherein R is a hydrogen atom or a methyl radical, with a solid component which comprises one or more polymers selected from the group consisting of an acrylate polymer, a methacrylate polymer and a copolymer of these polymers, and
initiating the polymerisation of the one or more monomeric esters in the liquid component,
characterised in that R' is

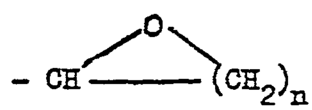

wherein n is 1, 2, 3 or 4 and further in that said one or more monomeric esters are the sole polymerisable constituent of the liquid component.

2. A process according to claim 1 characterised in that the liquid component contains, as its sole polymerisable constituent, tetrahydrofurfuryl methacrylate.

3. A process according to either claim 1 or claim 2 characterised in that the solid component comprises one or more polymers selected from the group consisting of polymethyl methacrylate, polyethyl methacrylate, poly propyl methacrylate, polybutyl methacrylate or copolymers of two or more of these polymers.

4. A process according to claim 3 characterised in that the solid component comprises polyethyl methacrylate or a copolymer of polyethyl methacrylate with one of polymethyl methacrylate, polypropyl methacrylate or polybutyl methacrylate.

5. A process according to any one of claims 1 to 4 characterised in that the polymerisation of the one or more monomeric esters in the liquid component is initiated by a free radical catalyst, preferably benzoyl peroxide, present in the solid component.

6. A process according to claim 5 characterised in that the free radical catalyst is activated by a chemical activator, selected from the group consisting of N, N - dialkyl - p-toluidines, N, N - dialkyl anilines and N - p - tolyldialkanolamines, preferably N, N - dimethyl - p - toluidine, N,N - diethyl - p - toluidine, N, N - dimethylaniline and N - p - toluene diethanolamine, present in the liquid component.

7. A polymeric material suitable for biomedical use and having a linear curing shrinkage of 1% or less whenever prepared by a process according to any one of claims 1 to 6.

8. A polymeric material according to claim 7 in the form of a biomedical appliance.

9. A polymeric material according to claim 8 in the form of a hearing aid, a tooth filling, a dental bridge or crown, dentures, an artificial eye, a contact lens or a bone cement.

10. A kit for use in the preparation of a polymeric material by a process according to claim 1 comprising

(a) a liquid component which contains one or more monomeric esters of general formula I

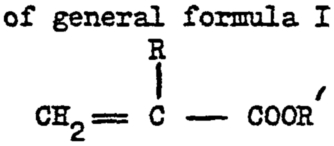

I

wherein R is a hydrogen atom or a methyl radical, and

(b) a solid component which comprises one or more polymers selected from the group consisting of an acrylate polymer, a methacrylate polymer and a copolymer of these polymers

characterised in that $R'$ is 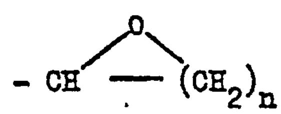

wherein n is 1, 2, 3 or 4 and further in that said one or more monomeric esters are the sole polymerisable constituent of the liquid component.

11. A kit according to claim 10 further comprising a free radical catalyst in the solid component and a chemical activator of the free radical catalyst in the liquid component.

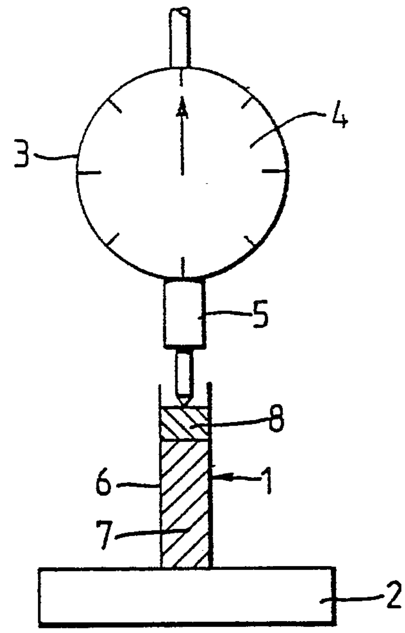
3
4
5
8
6
1
7
2

Fig. 1

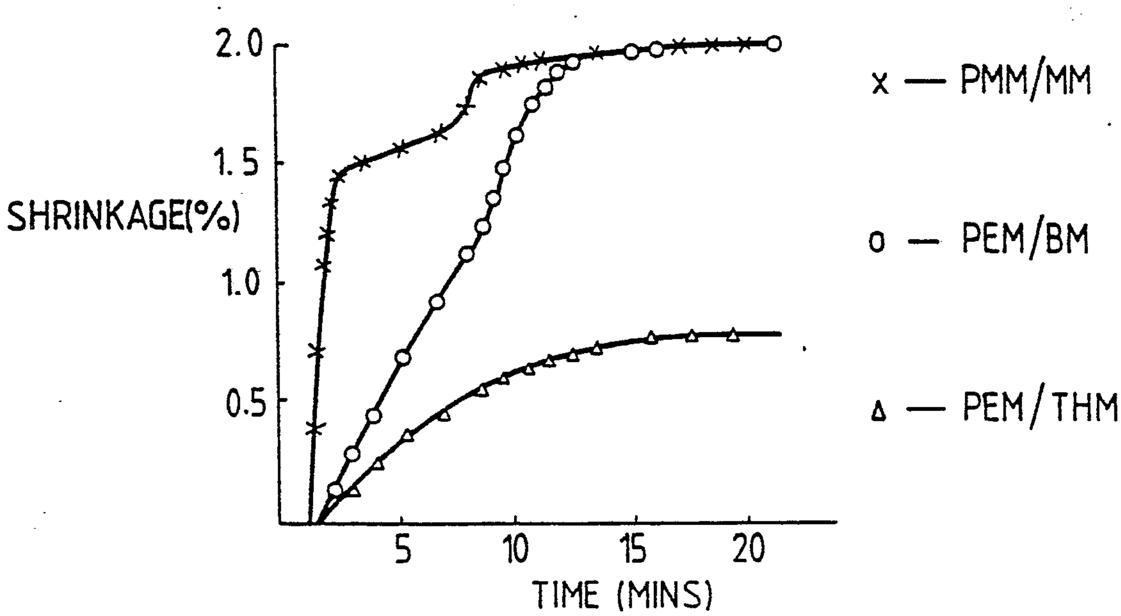
2.0
1.5
SHRINKAGE(%)
1.0
0.5
5
10
15
20
TIME (MINS)
x — PMM/MM
o — PEM/BM
Δ — PEM/THM

Fig. 2